Europäisches Patentamt

European Patent Office

Office européen des brevets

(19)

(11) Publication number: **0 029 708**

**A1**

(12) **EUROPEAN PATENT APPLICATION**

(21) Application number: **80304148.2**

(22) Date of filing: **20.11.80**

(51) Int. Cl.³: **A 61 F 5/46**
**A 61 F 13/20, A 61 L 15/00**

(30) Priority: **21.11.79 US 96295**

(43) Date of publication of application:
**03.06.81 Bulletin 81/22**

(84) Designated Contracting States:
**AT BE CH DE FR GB IT LI LU NL SE**

(71) Applicant: **VORHAUER LABORATORIES, LTD.**
**130 McCormick Avenue**
**No. 104, Costa Mesa California 92626(US)**

(72) Inventor: **Vorhauer, Bruce Ward**
**17902 Butler**
**Irvine California 92715(US)**

(72) Inventor: **Dobbie, Thomas Auther, Jr.**
**2662 Vista Drive**
**Newport Beach California 92660(US)**

(74) Representative: **McCall, John Douglas et al,**
**W.P. THOMPSON & CO. Coopers Building Church Street**
**Liverpool L1 3AB(GB)**

㊄ Biologically compatible tampon contraceptive and method of forming same.

㊄ The biocompatible tampon contraceptive (10) comprises a soft, flexible, porous polymeric sponge body (12), one side of said body having a recess (16) contoured to fit over the os of the cervix is disclosed. A removal loop (18) may be attached to the sponge to aid in removal of the sponge from the vaginal canal.

Fig. 1

EP 0 029 708 A1

Croydon Printing Company Ltd.

## DESCRIPTION

"BIOLOGICALLY COMPATIBLE TAMPON CONTRACEPTIVE AND
METHOD OF FORMING SAME"

The present invention relates to a biologically compatible tampon contraceptive and to a method of forming same.

Social scientists recognize that overpopulation is one of the most serious problems which mankind must face and solve. As an example, in the United States, the Department of Health, Education and Welfare released statistics recently which showed that 3.3 million babies were born in the United States in 1977, up 5% from the previous year. This marked an increase in the nation's birthrate for the first time since 1970. The problem is even more critical in underdeveloped countries.

In order to control our increasing world population, there exists a myriad of contraceptive devices to prevent unwanted pregnancies. Each method attempts to achieve a contraceptive possessing the qualities of simplicity, acceptability, efficacy, non-toxicity, and an absence of adverse side effects (bicompatibility).

One class of such measures are mechanical contraceptives, such as the diaphragm, which are inserted into the vagina to occlude the os of the cervix, thus obstructing the migration of sperm upward into the uterus and fallopian tubes. Such contraceptives have serious application problems due to the wide variation in size and geometry of the vaginal canal and the cervical opening. These devices often require special insertion instruments and careful fittings, usually by a trained physician. Furthermore, since trained help and application is often necessary, these devices are ill-suited for the under-developed countries where they are needed most.

Another type of mechanical device is the condom, a rubber device that surrounds the penis and contains the sperm after ejaculation. This widely used device suffers from an unnatural or desensitizing feeling to the male and female. In addition, the possibility of a perforation in the sheath makes the device less than the ultimate answer to unwanted pregnancy.

According to the present invention there is provided a contraceptive device and method of manufacture which will obviate the deficiencies in the prior art. The device is a soft, flexible, porous polymeric sponge which is generally in the shape of a flattened ball. One side of the sponge has a recess which is contoured to fit over the os of the female cervix. Thus, when the sponge is inserted into the vaginal canal, the cervical opening is positioned within the recess in the contraceptive sponge ball. This allows the sponge to be inserted as deeply as possible into the vaginal canal and allows the sponge to effectively cover and block the cervical opening from receiving sperm. The sponge, inserted more deeply into the vaginal canal, is not felt by the male or female during intercourse.

Since the sponge is made from a soft, pliable polymer, the sponge easily adapts itself to the size of the vaginal canal and to the cervical opening. The product is a non-prescription device and can be inserted in privacy without a special applicator or counsel of a physician. Since the sponge is also porous, it not only acts as an effective barrier to sperm, but also acts to absorb sperm thereby decreasing the number of sperm available for fertilization.

The sponge is also equipped with a removal loop which aids in removal of the sponge from the vaginal canal. The recess also functions to aid in removal because the sponge has a tendency to fold over itself, thereby reducing its volume.

Optionally, a spermicide can be added to the sponge so that the sponge not only acts as a mechanical barrier, but will chemically act to immobolize sperm by slowly releasing a spermicide.

The disclosed invention therefore is a contraceptive device possessing all of the following properties:

1.  A simple contraceptive which can be inserted in privacy without a special applicator or counsel of a physician (non-prescription product).

2.  A biocompatible, non-toxic, non-irritating contraceptive with no adverse side effects.

3.  A soft, flexible contraceptive which easily adapts itself to the size of the vaginal opening and cervical opening.

4.  A contraceptive which acts as a barrier to the sperm and also absorbs the sperm.

5.  A contraceptive which is contoured to fit over the cervical opening, thereby allowing the sponge to be inserted as deeply as possible into the vaginal canal.

6.  A contraceptive which can be inserted deeply into the vaginal canal, is natural and soft in feeling, and which neither sexual partner is aware of during intercourse.

7.  A contraceptive which is reusable and can be easily removed from the vaginal canal.

8.  A contraceptive which can include a spermicide which provides long lasting contraceptive effectiveness, a factor of immense importance in underdeveloped countries.

These advantages, and other novel features, which are believed to be characteristic of the invention, will be better understood from the following description considered in connection with the accompanying drawings in which a presently preferred embodiment of the invention is illustrated by way of examples.  It is to be expressly understood, however, that the drawings are for the purpose of illustration and description only, and are not intended as a definition of the limits of the invention.

A detailed description of the invention will be made with the aid of the accompanying drawings, wherein:

Figure 1 is a perspective view of the tampon sponge contraceptive;

Figure 2 is a schematic view of the sponge of Figure 1 positioned over the cervical opening;

Figure 3 is a schematic view of the sponge of Figure 1 during the process of removal from the vaginal canal; and

Figure 4 is a cross-sectional view of a two-cavity mold used to manufacture the invention.

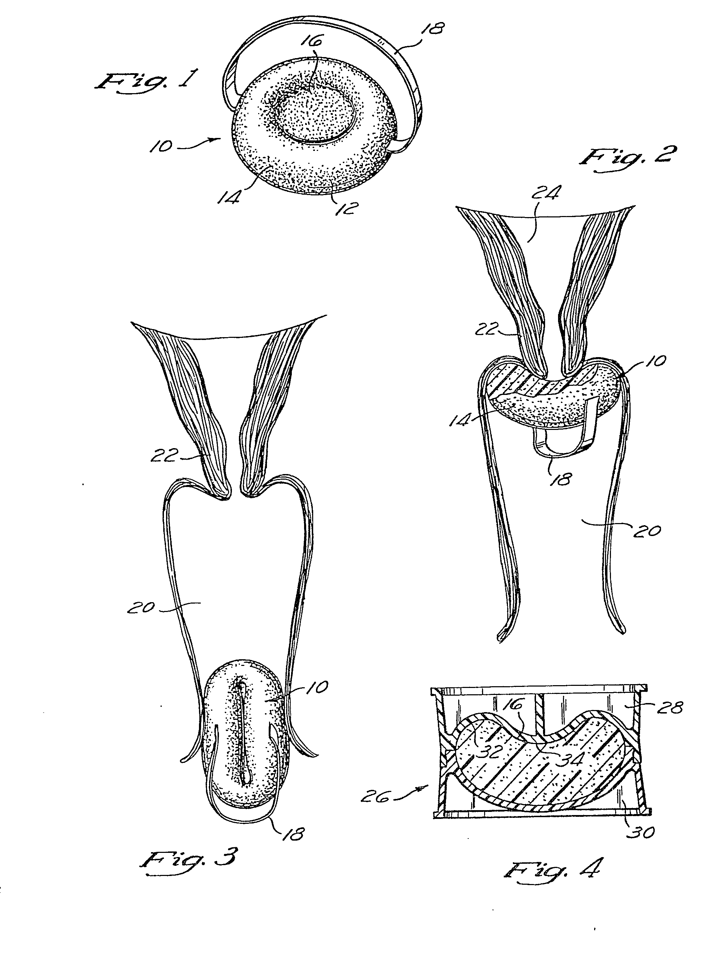

Referring to the drawings, Figure 1 illustrates a tampon sponge contraceptive 10. The sponge 10 has a soft, flexible, porous sponge body 12 which is generally in the shape of a dimpled flattened ball with a diameter of approximately 2 inches (approximately 5.1 cms). The sponge body 12 has tiny pores 14 which are in communication with the sponge's environment and which also extend throughout the sponge's inner structure (not shown). One side of the sponge body 12 has a recess 16. This recess or dimple 16 is generally circular, centrally located, and its depth increases from the perimeter of the recess toward its centre. The recess has an approximate diameter of 1 inch (2.54 cms). The thickness of the sponge at its largest point is approximately 1 inch. The thickness of the sponge at its smallest point, which would be approximately from the central area of the recess to the opposite side of the sponge is approximately 3/4 of an inch (approximately 1.9 cms). A flexible loop 18 is attached to the sponge body 12 to provide a convenient removal means.

Referring to Figure 2, the sponge 10 is shown inserted within the vaginal canal 20. The recess 16 is shown positioned over the cervical opening 22, which leads to the uterus 24. When positioned properly, the cervical opening 22 is positioned within the recess 16 so that the sponge 10 effectively covers and blocks the cervical opening from receiving sperm. Since the sponge 10 is contoured to fit over the cervical opening 22, the sponge 10 acts as a more effective barrier to sperm attempting to enter the uterus 24. The fact that the recess 16 also allows the sponge 10 to be inserted more deeply into the vaginal canal 20 over the cervical opening 22, means that there is less chance that the sponge 10 will be felt by either partner during sexual intercourse. Since the sponge 10 is made from a soft, flexible polymeric material, it can easily adapt itself to the varying size of the vaginal canal 20 and the cervical opening 22. Upon absorbing body fluids, the sponge also has a tendency to slightly expand in volume which also allows the sponge to more closely fit within the vaginal canal 20 and over the cervical opening 22. The pores 14 of the sponge also act to absorb sperm attempting to enter the uterus 24.

Thus, the sponge's unique composition and structure provide advantages and properties heretofore unknown in prior art devices. The soft and flexible nature of the sponge 10 allows it to more easily adapt to the size of the vaginal canal 20 and cervical opening 22. The recess 16 allows the sponge 10 to be inserted more deeply within the vaginal canal 20 and to effectively cover the cervical opening 22. The pores 14 of the sponge 10 allow it to effectively absorb sperm as well as acting as a barrier.

Referring to Figure 3, the sponge 10 is shown during the removal process. By grasping the loop 18, the user can easily remove the sponge 10 from the vaginal canal 20. Due to the soft, flexible nature of the sponge 10, and the recess 16, as the sponge 10 is removed, it has a tendency to fold over itself, as shown in Figure 3. This reduces the volume of the sponge 10 and makes it easier to remove from the vaginal canal 20.

The sponge 10 is formed from a polymer mixture which is foamed within a two-cavity mold, shown generally as 26 in Figure 4. Referring to Figure 4, two halves of the mold 26 are shown as upper half 28 and lower half 30. The cavity of the mold 32 is complementarily shaped to produce the sponge 10 and includes a protruding portion 34, which produces the recess 16 of the sponge 10.

The material and surface finish of the mold are important in several respects. The surface of the mold is critical in forming the exterior characteristics of the sponge. For example, in forming the sponge 10 upon foaming, there is a tendency for the sponge 10 to form a shiny skin-like surface on its exterior. This is quite undesirable for the reasons that the sponge 10 will not absorb the sperm as well nor will it release spermicide through its outer pores as well. It has now been discovered that polypropylene or polyallomer are acceptable polymers in formation of the mold 32. The polyallomer which is a polymer manufactured by Eastman Chemical Products, Inc., Plastics Division, Kingsport, Tennessee, has been found to be especially advantageous particularly in view of the fact that it is generally recognized as safe as used in intravenous solution bottles. Both. the polypropylene and polyallomer have been found to be successful in preventing any skin formation on the contraceptive sponge 10.

Secondly, and probably more important than the actual material of the mold 32, the surface finish of the mold 32 must not be smooth. A smooth finish will produce a sponge with a shiny skin. However, a certain finish which is about 60 grit, i.e., the finish resulting from use of a 60-grit sandblast, provides a rough finish which will prevent any skin formation.

The polymer solution is poured into the two halves of the mold 26. The foam forms by spontaneous exothermic reaction filling the mold cavity 32. Just before the mold 26 is closed, a soft woven polyester ribbon loop 18, as shown in Figures 1 to 3, is laid over one half of the mold 28, and the other half 30 is quickly closed over half 28 with the loop inbetween.

With the mold closed, the polymer continues to foam with the air escaping out through a crack around the middle of the mold, so that no air bubbles are trapped in the sponge. The shot size of material poured into the mold is measured precisely so that the sponge will just fill the container. The air escapes through the mold seam as the foam blows, but the polymer solution will not escape, since it is more viscous than the air. Shortly after the foaming operation is completed, the sponge 10 is placed in a sealed bag for product protection and patient labeling and instructions. The product is now ready to be commercially sold. The loop 18 on the sponge 10 allows the contraceptive to be easily grasped for removal from the vaginal canal 20. The removal loop 18 is made from 100% woven polyester, which is a soft weave, non-irritating, and biocompatible. The loops 18 can be welded into a circular shape with an ultrasonic weld or can simply be overlapped, allowing the sponge 10 to encapsulate and bond the overlapped ends.

The preferred prepolymer used is a polyurethane prepolymer which is water catalyzed with large amounts of water. More specifically, the preferred prepolymer is an isocyanate capped polyoxyethylene polyol. A comprehensive description of this prepolymer polyol is given in U.S. Patent No. 3,903,232, issued to Wood et al, which is hereby incorporated by reference.

In a preferred embodiment, a spermicide is added to the solution of water which is, in turn, added to the prepolymer. To effect foaming and preparation of the sponge structure, a solution of water containing various additives, including the spermicide, is mixed with a solution of the urethane prepolymer. The ratio of polymer to water mixture is in the range of 30 to 150 parts by weight polymer to 100 parts by weight water mixture. Conversely, the ratio of water mixture to polymer is 66 to 133 parts by weight water mixture to 100 parts by weight polymer. If either an excess or a deficiency of water is present, an incomplete reaction results. In the preferred embodiment, 77.77 parts of the polymer are mixed with 100 parts water mixture in order that the volume ratios would result with two volumes of water mixture to one volume of polymer. This 2 to 1 volume ratio, which is exemplary only, was chosen for convenience in order that in the manufacturing process, two units of water mixture could be added to one unit of polymeric solution.

The final optimum ratios in parts by weight are:

4.5 parts water;

1.5 parts spermicide; and

3.5 parts urethane prepolymer.

This gives the above-indicated volume ratio for the water to prepolymer of 2 to 1.

The preferred spermicide is nonylphenoxypoly (ethyleneoxy) ethanol, herein referred to as nonoxynol 9, which is a spermicide commonly used in vaginal birth control foams, gels, and creams. Other examples of such spermicide surfactants which should be equivalent are p-menthanyl phenylpolyoxyethylene ether, nonyl phenylpolyexylethylene ether, octyl cresolpolyoxyethylene ether, polyoxyethylene oxypropylene stearate, polyoxyethylene laurate, glycerol ricinolate, di-iso-butyl phenylpolyoxyethylene ether, tri-isopropyl phenylpolyoxyethylene ether, mono-iso-octyl phenyl 500 laureate, polyoxyethylene stearylamine, benzalconium chloride, cetyl trimethylammonium bromide, methyl benzetonium chloride, benzetonium chloride, sodium dodecylsulfate, di-2-ethylhexyl sodium sulfosuccinate, nonylphenolpolyethylene sodium sulfate, sodium oleate, zinc phenolsulfonate, dodecylbenzen sulfonate, dodecyl diaminoethyl-glycine and the like.

The acceptable minimum percentage of spermicide in the sponge is greater than about 10% dry weight of the sponge, preferably above about 20%, and optimally 30%. The formulation of these percentages is a highly surprising discovery and resulted from rigorous experimentation. The high percentages of spermicide/surfactant present in the claimed invention are unusual because heretofore it was well known that in producing polymer foams, it is desirable to use as little surfactant as possible in order to maintain
the purity of the resulting polymeric foam.  However,
it was found that the use of these surfactants in
percentages of 10% or less produced foams which did
not retain the spermicide for any appreciable period.
The spermicides were quickly washed out of the sponge.
It has now been discovered that the surfactant
concentration has to be increased in order to decrease
the polymeric cellular foam size thereby increasing
the sponge's capacity to retain the spermicide.

It was first thought, as described in co-pending
application, Serial No. 900,864, that the maximum
percentage of spermicide which could be used was
approximately 50%.  The reason for this was that the
sponge was thought to become too viscous and sticky to
produce an acceptable sponge.  However, after
additional experimentation, it has been learned that
the percentage of spermicide can be increased to
greater levels.  For example, sponges of approximately
75% spermicide have been produced, although the sponge
tends to become soft and viscous and tends to adhere to
the mold.  The maximum percentage of spermicide is
therefore defined at that level above which the sponge
will not properly cure, since it simply contains an
excess of the viscous spermicide vis-a-vis the polymer.
This maximum range is therefore approximately 70 to
80% spermicide.

By increasing the dry weight percentages of
spermicide to preferably around 30% or above, an
extraordinary amount of spermicide is available in
the sponge and the cellular pore size in the sponge
is decreased sufficiently to produce a sponge with
a great capacity to retain the spermicide.  This
decrease in cellular pore size in the sponge has made it unnecessary to include an additive, such as collagen, which is quite an important result, since collagen prevents several problems from a quality control standpoint, i.e., purification, etc.

Several additives may optionally be added to the water solution in addition to the spermicide. For example, various preservatives, anti-fungicides, antibacterial agents, and anti-oxidants may be added. Also, pH adjusters and buffers may be added. In the preferred embodiment, distilled water is mixed with 1/10 of 1% of benzoic acid, 2/10 of 1% sorbic acid, 1/20 of 1% of sodium hydroxide, 1/2 of 1% monohydrate citric acid, and 5/100 of 1% sodium metabisulfite. More specific details relating to the preparation of the sponge 10 can be found in co-pending application, Serial No. 900,864 and concurrently filed application Serial No. 096,293 entitled "Biologically Compatible Tampon Contraceptive", which are hereby incorporated by reference.

As stated earlier, in a preferred embodiment, two volumes of the water mixture solution are mixed with one volume of the urethane prepolymer. This mixture is effected by a high shear mixer which produces a creaming action and the solution is ready to be poured or injected into the mold 26 to begin its forming action. The prepolymer can optionally be heated to temperatures of approximately 110 to 120°F (43.3 to 48.9°C). These higher temperatures lower the cell density of the resulting sponge making the resulting sponge more porous and easier to handle.

The following examples are set forth in order to clearly point out the detailed description of the invention but are not intended as a limitation to the same. Throughout the specification and the following examples, all parts are expressed in parts by weight unless otherwise indicated.

-12-

### Example 1

8 parts urethane prepolymer were mixed with a solution consisting of 2 parts nonoxynol 9 surfactant/spermicide and 8 parts of water. This produced a sponge with fine cell structure and a nonoxynol dry weight content of 20%.

### Example 2

18 parts of the urethane prepolymer were mixed with a solution consisting of 2 parts nonoxynol 9 surfactant/spermicide and 16 parts water. This produced a sponge which had a nonoxynol dry weight content of 10% and gave a sponge which was soft and flexible but that did not retain the spermicide to a fully acceptable degree.

### Example 3

8 parts of the urethane prepolymer were mixed with a solution consisting of 2 parts nonoxynol 9 surfactant/spermicide and 5 parts water. This gave a 20% by dry weight sponge of spermicide and also gave a sponge of good fine cell structure with a lower weight and more resilience than a sponge produced from equal weights of water and polymer.

### Example 4

The urethane prepolymer in Example 3 was heated to about $110^{\circ}F$ ($43.3^{\circ}C$) and there was more foaming of the polymer and a sponge resulted which had slightly larger cells and lower cellular density than the sponge in Example 3.

### Example 5

16 parts of the urethane prepolymer were added to a solution consisting of 4 parts of nonoxynol 9 surfactant/spermicide and 14 parts water and 1/10 of 1% USP benzoic acid as a preservative. This produced a sponge of good quality and clinically acceptable.

-13-

## Example 6

16 parts of the urethane prepolymer were added to a solution consisting of 4 parts nonoxynol 9 surfactant/spermicide and 12 parts water and 1/10 of 1% benzoic acid added to the water. The resulting sponge was of somewhat lesser weight than the sponge of the previous example and had a slightly finer pore size.

## Example 7

16 parts of the urethane prepolymer were added to a solution consisting of 4 parts of nonoxynol 9 surfactant/spermicide and 12 parts water and .16 parts zinc sulphate. This gave a sponge of good quality and was clinically acceptable.

## Example 8

14 parts of the urethane prepolymer were added to a solution consisting of 6 parts of nonoxynol 9 surfactant/spermicide, 12 parts water, and .16 parts zinc sulphate. These sponges were acceptable although the cell size was somewhat larger because of the zinc sulphate.

## Example 9

A 50% nonoxynol 9 sponge was produced from mixing 12 grams of nonoxynol 9, 12 grams of water, and 12 grams of the urethane prepolymer 2,001 Hypol (registered trademark) polymer, manufactured by W. R. Grace & Co. The resulting sponge was a commercially acceptable, nice soft sponge.

## Example 10

A 50% nonoxynol 9 sponge was produced from mixing 12 grams of nonoxynol 9, 12 grams of water, and 12 grams of urethane prepolymer 3,001 Hypol polymer, manufactured by W. R. Grace & Co. The resulting sponge was commercially acceptable but somewhat stiff.

-14-

## Example 11

A 60% nonoxynol 9 sponge was produced from mixing 3 parts nonoxynol 9, 2 parts water, and 2 parts urethane prepolymer 2,001 Hypol polymer, manufactured by W. R. Grace & Co. The resulting sponge had a sticky surface but was fully blown.

## Example 12

A 60% nonoxynol 9 sponge was produced from mixing 3 parts nonoxynol 9, 2 parts water, and 2 parts 3,001 Hypol polymer. The resulting sponge was good and commercially acceptable.

## Example 13

A 65% nonoxynol 9 sponge was made from 6.5 grams nonoxynol 9, 4.5 grams water, and 3.5 grams 2,001 Hypol polymer. This sponge took a very long time to cure.

## Example 14

A 70% nonoxynol 9 sponge was made from 3 grams water, 7 grams nonoxynol 9, and 3 grams 3,001 Hypol polymer. 8 grams of this mixture were poured into the mold which resulted in a very soft sponge which stuck to the mold.

## Example 15

A 75% nonoxynol 9 sponge was made from 18 grams nonoxynol 9, 6 grams water, and 6 grams 2,001 Hypol polymer. The resulting sponge was mushy, gooey, and almost not cured.

# CLAIMS.

1. A biocompatible tampon contraceptive (10) characterized by comprising a soft, flexible, porous polymeric sponge body (12), said body being generally in the shape of a flattened ball, one side of said body having a recess (16) contoured to fit over the os of the cervix.

2. A biocompatible tampon contraceptive as claimed in claim 1, characterized in that it also comprises a removal loop attached to said sponge to aid in the removal of said sponge from the vaginal canal.

3. A biocompatible tampon contraceptive as claimed in claim 1 or claim 2, characterized in that said sponge contains a spermicide.

4. A biocompatible tampon contraceptive as claimed in claim 3, characterized in that said spermicide is nonylphenoxypoly(ethyleneoxy)ethanol.

5. A biocompatible tampon contraceptive as claimed in claim 3 or 4, characterized in that said spermicide comprises at least about 10% by dry weight of the sponge.

6. A biocompatible tampon contraceptive as claimed in claim 5, characterized in that said spermicide comprises at least about 20% by dry weight of the sponge.

7. A biocompatible tampon contraceptive as claimed in any of claims 1 to 6, characterized in that said polymer sponge body is formed from a urethane polymer.

8. A method for forming a biocompatible tampon contraceptive sponge characterized by the steps of: placing a foam forming prepolymer mixture into a mold; and permitting said mixture to foam in said mold and form a soft, flexible porous sponge, said sponge being formed by said mold into the shape of a flattened ball with one side of said sponge having a recess contoured to fit over the os of the cervix.

9. A method as claimed in claim 8, characterized in that said prepolymer mixture contains a spermicide.

10. A method as claimed in claim 9, characterized in that said spermicide is nonylphenoxypoly(ethyleneoxy)ethanol.

11. A method as claimed in claim 9 or claim 10, characterized in that said spermicide comprises at least about 10% by dry weight of the sponge.

12. A method as claimed in claim 11, characterized in that said spermicide comprises at least about 20% by dry weight of the sponge.

13. A method as claimed in any of claims 8 to 12, characterized in that said prepolymer mixture contains a urethane prepolymer.

Fig. 1

Fig. 2

Fig. 3

Fig. 4

European Patent Office

**EUROPEAN SEARCH REPORT**

Application number

EP 80 30 4148.2

| Category | Citation of document with indication, where appropriate, of relevant passages | Relevant to claim |
|---|---|---|
| X | DE - C - 822 877 (GOOSSENS)<br>* claim 1; fig. 1 *<br>-- | 1-3 |
| X | DE - U - 6 933 451 (GOOSSENS)<br>* claims 1, 3; fig. 1, 2 *<br>-- | 1-3 |
| X | FR - A - 1 189 918 (VINTRE)<br>* fig. 1 to 5 *<br>-- | 1 |
|  | WO - A1 - 79/00014 (VORHAUER LABORA-<br>TORIES)<br>* entire document *<br>-- | 1-13 |
|  | DE - A1 - 2 716 386 (CHVAPIL)<br>* claims 1, 9; fig. 1 to 3 *<br>-- | 1,8 |
|  | FR - A1 - 2 295 731 (CHAMPION et al.)<br>* fig. *<br>-- | 1,2 |
|  | US - A - 3 918 452 (CORNFELD)<br>* claims 1, 3 *<br>-- | 1-3,7 |
|  | US - A - 3 128 762 (YOUNG)<br>* claim 1; fig. 1 *<br>-- | 1,7 |
| A | DE - U - 1 609 913 (BAUER)<br>* fig. 2 *<br>--<br>./.. | 1 |

**DOCUMENTS CONSIDERED TO BE RELEVANT**

CLASSIFICATION OF THE
APPLICATION (Int. Cl.³)

A 61 F    5/46
A 61 F   13/20
A 61 L   15/00

TECHNICAL FIELDS
SEARCHED (Int. Cl.³)

A 61 F    5/00
A 61 F   13/00
A 61 K    9/00
A 61 L   15/00

CATEGORY OF
CITED DOCUMENTS

X: particularly relevant
A: technological background
O: non-written disclosure
P: intermediate document
T: theory or principle underlying
   the invention
E: conflicting application
D: document cited in the
   application
L: citation for other reasons

&: member of the same patent
   family,
   corresponding document

The present search report has been drawn up for all claims

| Place of search | Date of completion of the search | Examiner |
|---|---|---|
| Berlin | 12-02-1981 | KANAL |

EPO Form 1503.1  06.78

| Category | DOCUMENTS CONSIDERED TO BE RELEVANT | Relevant to claim | CLASSIFICATION OF THE APPLICATION (Int. Cl.³) |
|---|---|---|---|
| | Citation of document with indication, where appropriate, of relevant passages | | |
| A | US - A - 2 020 107 (CRUICKSHANK)<br>* claim 1; fig. 1 * | 1 | |
| A | US - A - 1 480 680 (GLOVER)<br>* fig. 1 * | 1 | |
| P | WO - A1 - 80/00008 (DONALD ENTERPRISES)<br>* claims 1, 2, 7 * | 1-3,7 | |
| | | | TECHNICAL FIELDS SEARCHED (Int. Cl.³) |

EPO Form 1503.2   06.78